# EUROPÄISCHE PATENTSCHRIFT

(11) **EP 0 486 422 B1**
(45) Veröffentlichungstag und Bekanntmachung des Hinweises auf die Patenterteilung: **25.01.1995**
(21) Anmeldenummer: 91810792.1
(22) Anmeldetag: 15.10.1991
(51) Int. Cl.: A61F 2/36, A61F 2/30

(54) **Verankerungsschaft für eine Femurkopfprothese**
Anchoring shaft for a femoral head prosthesis
Tige d'ancorage pour une prothèse de tête fémorale

(30) Priorität: 16.11.1990 CH 3645/90
(43) Veröffentlichungstag der Anmeldung: 20.05.1992
(73) Patentinhaber: SULZER Medizinaltechnik AG, 8404 Winterthur (CH); PROTEK AG, 3110 Münsingen-Bern (CH)
(72) Erfinder: Spotorno, Lorenzo, Prof. Dr.-med., I-17024 Finale Ligure (IT); Frey, Otto, Dr., CH-8400 Winterthur (CH)
(74) Vertreter: Trieblnig, Adolf

(56) Entgegenhaltungen:
- EP-A- 0 189 546
- EP-A- 0 234 358
- EP-A- 0 359 672
- EP-A- 0 404 716
- FR-A- 2 305 961
- FR-A- 2 633 510

## Beschreibung

Die Erfindung betrifft einen Verankerungsschaft für eine Femurkopfprothese, bei dem unterhalb des den Schaft vom Prothesenhals trennenden Kragens von medial her ein Hohlraum vorhanden ist, der durch einen Kunststoff-Füllkörper ausgefüllt ist.

Ein Verankerungsschaft der vorstehend genannten Art ist bekannt aus der CH-Anmeldung 02 316/89-6 (EP-A-0 404 716). Der mediale Schlitz und der kunststoffgefüllte Hohlraum haben die Aufgabe, Wechselbelastungen an dem auf dem Knochengewebe aufliegenden Kragen zu vermeiden. In der Praxis haben sich bei dieser Konstruktion Unzulänglichkeiten gezeigt. Die offenen Seiten des Hohlraumes ermöglichen bei Druckbelastungen ein "Herausquellen" des Kunststoff-Füllkörpers aus dem Schaft. Dies führt zum einen zu unzulässig langen "Federwegen" des Prothesenoberteils mit dem Kragen relativ zum im Knochen verankerten Prothesenschaft; zum anderen führen die wechselnden Be- und Entlastungen der Prothese zu Kunststoffabrieb, der in das Knochengewebe gelangen kann. Durch die Kontakte mit dem lebenden Gewebe oder einem Knochenzement kann darüberhinaus der Kunststoff des Füllkörpers im Laufe der Zeit beeinflusst werden, wodurch unter Umständen seine Eigenschaften nachteilig verändert werden.

Aufgabe der Erfindung ist es, diese Unzulänglichkeiten der bekannten Konstruktion zu vermeiden. Dies geschieht dadurch, dass der Hohlraum von einer Abdeckung umschlossen ist.

Durch die Abdeckung wird einerseits die "Ausweichmöglichkeit" des Kunststoff-Füllkörpers verringert, so dass die Bewegung des Prothesenkopfes bzw. -halses gegenüber dem Schaft begrenzt wird, wobei die bei dieser Begrenzung auftretende Dämpfung umso grösser ist, je länger der Weg ist. Andererseits bewirkt die Abdeckung, dass praktisch keine direkten Kontakte des Kunststoff-Füllkörpers mit den Knochen oder einem Knochenzementbett gegeben sind.

Im folgenden wird die Erfindung anhand eines Ausführungsbeispiels im Zusammenhang mit der Zeichnung näher erläutert.
- Fig. 1: zeigt eine Ansicht des proximalen Teils des neuen Schaftes in einer gegenüber der natürlichen Grösse vergrösserten Darstellung;
- Fig. 2: ist ebenfalls im vergrösserten Massstab ein Schnitt II-II von Fig. 1.

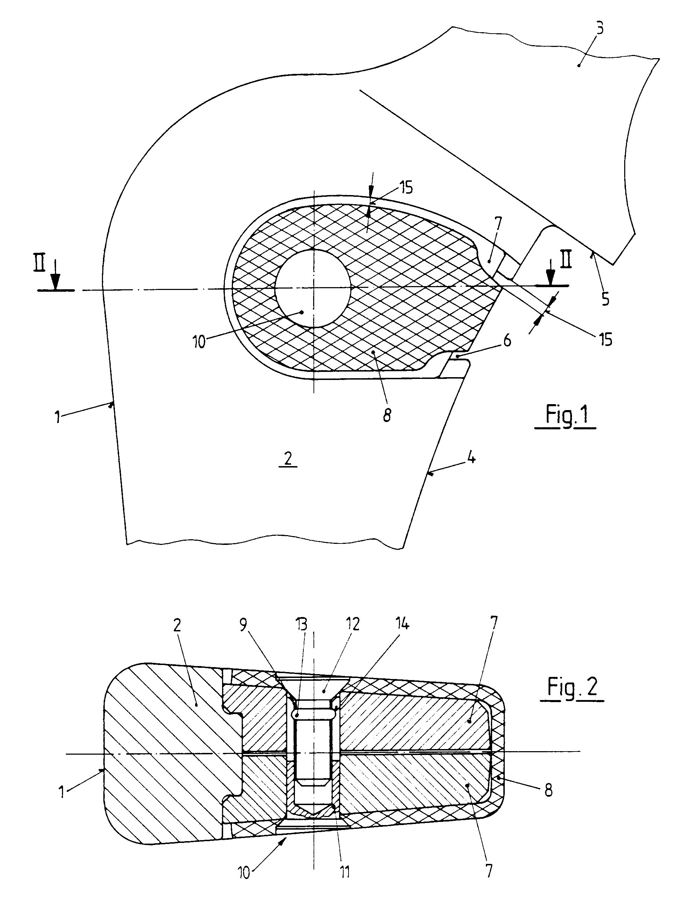

Die laterale Schmalseite 1 (Fig. 1) des Schaftes 2, der aus Metall, beispielsweise einer Titanlegierung, besteht und sich von seinem distalen Ende konisch erweitert, geht proximal über einen Kreisbogen zunächst in die Horizontale über und mündet dann in einen Prothesenhals 3; dieser trägt seinerseits einen nicht gezeigten konischen Zapfen, auf den ein ebenfalls nicht dargestellter Gelenkkopf aufsteckbar ist.

Die mediale Schmalseite 4 des Schaftes 2 verläuft in einer stetigen Kurve und endet an einem Kragen 5, der den Prothesenhals 3 von dem eigentlichen Schaftkörper 2 trennt. Die Grundfläche des Kragens 5 bildet eine Abstützfläche, mit der sich der Kragen 5 auf dem kortikalen Gewebe des nicht dargestellten Femurknochens abstützt; sie kann mit einer ein Anwachsen des Knochens fördernden Struktur versehen sein.

Wenige Millimeter unterhalb des Kragens 5 weist der Schaftkörper 2 von der medialen Schmalseite 4 her einen ballonähnlichen Hohlraum 6 auf, der von einem hoch elastischen Füllkörper 7 ausgefüllt ist, der im vorliegenden Beispiel aus Polyurethan oder Polyäthylen besteht.

Aus Montagegründen ist der Füllkörper 7 zweiteilig; er ist von einer U-förmigen Abdeckung 8 aus Metall umgeben, die in dem gezeigten Beispiel aus einem mehrlagigen Drahtnetz besteht, in dessen Maschen Knochen einwachsen oder Knochenzement eindringen kann. Selbstverständlich kann die Abdeckung 8 auch von einem Blech gebildet werden, das darüberhinaus ebenfalls mit einem den Kontakt zum Knochen fördernden Drahtnetz belegt sein kann.

Die Abdeckung 8 ist in der Oberfläche des Schaftes versenkt; um Relativbewegungen zwischen Prothesenkopf und -hals 3 einerseits und dem Schaft 2 andererseits zu ermöglichen, ist zwischen der Abdeckung 8 und der Begrenzung des Hohlraumes 6 im Schaft 2 längs des ganzen Umfanges der Abdeckung 8 ein Spiel 15 vorhanden.

Die Abdeckung 8 und der Kunststoff-Füllkörper 7 haben eine sich in Richtung ventral/dorsal erstreckte Bohrung 9, in die ein die Elemente 7 und 8 zusammenhaltender Schnappverschluss 10 eingesetzt ist. Der Verschluss 10 besteht im gezeigten Beispiel aus einer einseitig mit einem Kopf versehenen, am anderen Ende offenen Hülse 11, in die ein ebenfalls einen Kopf tragender Zapfen 12 eingepresst ist. Im Endzustand schnappt dabei ein Wulst 13 des Zapfens 12 in eine entsprechende rillenartige Vertiefung 14 im Innern der Hülse 11 ein.

## Patentansprüche

1. Verankerungsschaft (2) für eine Femurkopfprothese, bei dem unterhalb des den Schaft (2) vom Prothesenhals (3) trennenden Kragens (5) von medial her ein Hohlraum (6) vorhanden ist, der durch einen Kunststoff-Füllkörper (7) ausgefüllt ist, dadurch gekennzeichnet, dass der Hohlraum (6) von einer Abdeckung (8) umschlossen ist.

2. Verankerungsschaft nach Anspruch 1, dadurch gekennzeichnet, dass die Abdeckung (8) versenkt in die sie umgebende Schaftoberfläche eingebettet ist wobei zwischen ihr und dem Schaft (2) ein Spiel (15) vorhanden ist.

3. Verankerungsschaft nach Anspruch 1 oder 2, dadurch gekennzeichnet, dass die Abdeckung (8) aus einem mehrlagigen Metallnetz besteht.

4. Verankerungsschaft nach Anspruch 3, dadurch gekennzeichnet, dass das Metallnetz mit einem geschlossenen Blech unterlegt ist.

5. Verankerungsschaft nach einem der Ansprüche 1 bis 4, dadurch gekennzeichnet, dass die Abdeckung (8) U-förmig ausgebildet und von medial auf den Schaft (2) aufgeschoben ist.

## Claims

1. An anchoring shank (2) for a femur-head prosthesis, in which below the collar (5) separating the shank (2) from the neck (3) of the prosthesis there is a cavity (6) from the medial side, filled with a plastics filler (7), characterized in that the cavity (6) is enclosed by a covering (8).

2. An anchoring shank as in Claim 1, characterized in that the covering (8) is embedded below the surrounding surface of the shank, with a clearance (15) existing between it and the shank (2).

3. An anchoring shank as in Claim 1 or 2, characterized in that the covering (8) consists of a multilayer metal mesh.

4. An anchoring shank as in Claim 3, characterized in that closed sheetmetal underlies the metal mesh.

5. An anchoring shank as in one of the Claims 1 to 4, characterized in that the covering (8) is made U-shaped and pushed onto the shank (2) from the medial side.

## Revendications

1. Tige (2) d'ancrage d'une prothèse de tête de fémur, dans laquelle se trouve une cavité (6) pratiquée en partant du côté médial et qui est située sous le collet (5) séparant la tige (2) du col (3) de la prothèse et qui est remplie d'un corps de remplissage (7) en matière plastique, caractérisée en ce que la cavité (6) est enveloppée d'une chape (8).

2. Tige d'ancrage selon la revendication 1, caractérisée en ce que la chape (8) est noyée dans la surface de la tige qui l'entoure, un jeu (15) existant entre elle et la tige (2).

3. Tige d'ancrage selon la revendication 1 ou 2, caractérisée en ce que la chape (8) est formée d'un treillis métallique en plusieurs couches.

4. Tige d'ancrage selon la revendication 3, caractérisée en ce que le treillis métallique est doublé d'une tôle fermée.

5. Tige d'ancrage selon l'une des revendications 1 à 4, caractérisée en ce que la chape (8) a une forme en étrier et elle s'emboîte sur la tige (2) par le côté médial.
